(19) Europäisches Patentamt

European Patent Office

Office européen des brevets

(11) EP 1 410 814 A2

## (12) EUROPEAN PATENT APPLICATION

(43) Date of publication:
21.04.2004 Bulletin 2004/17

(51) Int Cl.$^7$: A61M 5/145

(21) Application number: 03023577.4

(22) Date of filing: 16.10.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 18.10.2002 JP 2002304057

(71) Applicant: Nipro Corporation
Kita-ku, Osaka-shi, Osaka-fu, 531-8510 (JP)

(72) Inventors:
• Mori, Takeshi
Osaka-shi Osaka-fu 531-8510 (JP)
• Hiejima, Katsuhiro
Osaka-shi Osaka-fu 531-8510 (JP)

(74) Representative:
Banzer, Hans-Jörg, Dipl.-Ing. et al
Kraus & Weisert
Patent- und Rechtsanwälte
Thomas-Wimmer-Ring 15
80539 München (DE)

## (54) Liquid medicine infusion apparatus

(57) A liquid medicine infusion apparatus including the following means (a) to (e):

(a) liquid medicine pressurizing/supplying means (1);
(b) a secondary pressurizing means (5) which is connected in liquid communication with the liquid medicine pressurizing/supplying means (1) by an upstream passage (2);
(c) upstreamopening/closingmeans (3) arranged in the upstream passage (2), for opening and closing the liquid communication state between the liquid medicine pressurizing/supplying means (1) and the secondary pressurizing means (5);
(d) downstream opening/closing means (4) arranged in a downstream passage (6) provided downstream of the secondary pressurizing means (5); and
(e) control means (7) for controlling the opening/closing timings of the upstream opening/closing means (3) and the downstream opening/closing means (4).

Fig. 1

## Description

Field of the Invention

The present invention relates to an infusion apparatus for administering a liquid medicine into a blood vessel, extradural cavity or hypodermically in small increments. Particularly, the invention relates to a liquid medicine infusion apparatus which can maintain a predetermined infusion rate stably for a long time without regard to the type of liquid medicine or the ambient temperature.

Background of the Invention

Among liquid medicines such as antibiotics and anti-cancer medicines and anesthetics, some liquid medicines are preferably administered into a blood vessel, extradural cavity or hypodermically little by little over a long time. Examples of known liquid medicine infusion apparatuses used for this purpose include a syringe pump type, in which the plunger of a syringe is pushed little by little by means such as a motor to thereby infuse the liquid medicine, and a roller pump type, in which a tube which is a conduit for a liquid medicine is slowly squeezed by a roller to force out the liquid medicine. Since these liquid medicine infusion apparatuses use electric power, they have an advantage that they can maintain an accurate infusion rate. However, because these liquid medicine infusion apparatuses generate the power for forcing out the liquid medicine by using electric energy, when one of the apparatuses is used for a long time, a large-sized battery needs to be used. Therefore, the infusion apparatus itself becomes heavy, making it very inconvenient for a patient to carry the infusion apparatus. In addition, such liquid medicine infusion apparatuses are complicated in structure and thus are expensive.

In view of the above drawbacks, there have also been proposed liquid medicine infusion apparatuses which do not use electric power. For example, Japanese Patent Unexamined Publication No. 2-11160 discloses a liquid medicine infusion apparatus in which a flow rate control unit composed of a tube having a small diameter is connected to liquid medicine pressurizing/supplying means such as a balloon. In this liquid medicine infusion apparatus, the infusion rate of a liquid medicine forced out from the balloon is controlled to a predetermined constant value due to the line resistance of the small-diameter inner tube while the liquid medicine passes through the flow rate control unit consisting of the small-diameter tube.

However, the control of the flow rate achieved by making use of the line resistance of the small-diameter tube is greatly influenced by the viscosity of the liquid medicine. That is, since the flow rate F of the liquid medicine after it passes through the small-diameter tube follows the Hagen-Poiseuille's law (see the following equation 1) , it is in inverse proportion to the viscosity $\eta$ of the liquid medicine.

$$F = \frac{gP\pi(d/2)^4}{8\eta l} \qquad (1)$$

F: flow rate of liquid medicine ($cm^3$/sec)
g: gravitational acceleration (980 cm/s)
P: pressure applied to liquid medicine (kPa)
d: inner diameter of small-diameter tube (cm)
$\eta$: viscosity of liquid medicine (g/cm·s)
l: length of small-diameter tube (cm)
n: ratio of circumference of circle to its diameter

Meanwhile, the viscosity of a liquid medicine becomes lower as the temperature rises according to the general properties of a fluid. Therefore, the flow rate of the liquid medicine increases in inverse proportion to the rise in the temperature of the liquid medicine. For example, the viscosity at 25°C of fluorouracil which is an anti-malignant antineoplastic agent is 1.273 g/cm·s whereas its viscosity at 32°C is reduced to 1.084 g/cm·s. Therefore, the flow rate at 32°C of fluorouracil is about 17.4% higher than that at 25°C.

The viscosity of a liquid medicine differs according to the type of the liquid medicine as well. For example, the viscosity at 25°C of fluorouracil is 1.273 g/cm·s whereas the viscosity at 25°C of cisplatin which is an anti-malignant tumor platinum complex is 0.898 g/cm·s. Therefore, the flow rate of a liquid medicine varies according to the type of the liquid medicine as well.

Thus, a liquid medicine infusion apparatus having a flow rate control unit, which consists of a small-diameter inner tube, connected to liquid medicine pressurizing/supplying means has a drawback in that a predetermined constant infusion rate cannot be obtained when the viscosity of a liquid medicine changes according to the type and temperature of the liquid medicine.

Therefore, conventionally known liquid medicine infusion apparatuses cannot be used for a long time without a large-sized battery or the liquid medicine infusion rate thereof is affected by the type and temperature of a liquid medicine.

It is an object of the present invention which has been made in view of the current state of the prior art to provide a liquid medicine infusion apparatus which does not have the drawbacks of conventionally known liquid medicine infusion apparatuses. Stated more specifically, it is an object of the present invention to provide a liquid medicine infusion apparatus which can be used for a long time without using a large-sized battery and can infuse a liquid medicine stably by maintaining a predetermined constant infusion rate without regard to the type and temperature of the liquid medicine.

According to the present invention, this object is achieved by a liquid medicine infusion apparatus as defined in claim 1. The dependent claims define preferred and advantageous embodiments of the invention.

## Summary of the Invention

The inventors of the present invention have conducted intensive studies to solve the above problems and have found that the above problems can be solved by controlling the flow rate with a novel mechanism without using a small-diameter tube as a flow rate control unit in a liquid medicine infusion apparatus which includes a liquid medicine pressurizing/supplying means and a flow rate control unit connected to the liquid medicine pressurizing/supplying means. The present invention has been accomplished based on this finding.

That is, the present invention relates to a liquid medicine infusion apparatus characterized by including the following means (a) to (e):

(a) liquid medicine pressurizing/supplying means;
(b) a secondary pressurizing means which is connected in liquid communication with the liquid medicine pressurizing/supplying means by an upstream passage;
(c) upstream opening/closing means arranged in the upstream passage, for opening and closing the liquid communication state between the liquid medicine pressurizing/supplying means and the secondary pressurizing means;
(d) downstream opening/closing means arranged in a downstream passage provided downstream of the secondary pressurizing means; and
(e) control means for controlling the opening/closing timing of the upstream opening/closing means and of the downstream opening/closing means.

According to another preferred embodiment of the present invention, the pressurizing means of the liquid medicine pressurizing/supplying means is a rubber elastic body, a spring, or air pressure.

According to still another preferred embodiment of the present invention, the pressurizing means of the secondary pressurizing means is a rubber elastic body, a spring, or air pressure.

According to yet still another preferred embodiment of the present invention, the upstream opening/closing means and the downstream opening/closing means are comprised of electromagnetic valves, clamps, or an integrated unit using a stopcock.

## Brief description of the Drawings

Fig. 1 is a schematic diagram of a liquid medicine infusion apparatus according to one embodiment of the present invention.
Figs. 2 (a) to (e) are diagrams for explaining the liquid medicine infusing operation of the liquid medicine infusion apparatus shown in Fig. 1.
Fig. 3 is a schematic diagram of a liquid medicine infusion apparatus according to another embodiment of the present invention.
Fig. 4 is a schematic diagram of a liquid medicine infusion apparatus according to still another embodiment of the present invention.
Fig. 5 is a schematic diagram of a liquid medicine infusion apparatus according to yet still another embodiment of the present invention.
Fig. 6 is a schematic diagram of a flow rate control unit of the liquid medicine infusion apparatus shown in Fig. 5 as viewed from above.
Fig. 7 is a schematic diagram of a liquid medicine infusion apparatus according to further still another embodiment of the present invention.
Figs. 8 (a) to (d) are diagrams for explaining the liquid medicine infusing operation of the liquid medicine infusion apparatus shown in Fig. 7.

## Preferred Embodiment of the Invention

The liquid medicine infusion apparatus of the present invention will be described with reference to the accompanying drawings. However, the present invention is not limited to the embodiments shown in the drawings, but includes all embodiments within the scope and sprit of the appended claims.

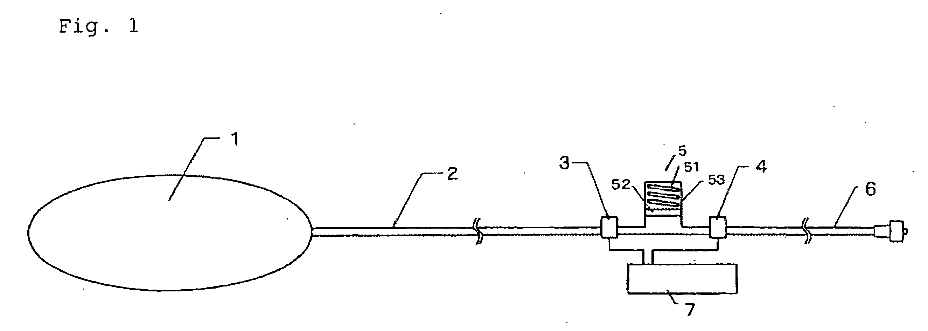

Fig. 1 is a schematic diagram of a liquid medicine infusion apparatus according to one embodiment of the present invention. The liquid medicine infusion apparatus of Fig.1 includes liquid medicine pressurizing/supplying means 1, secondary pressurizing means 5, upstream opening/closing means 3, downstream opening/closing means 4, and control means 7 for controlling the opening/closing timing of the upstream opening/closing means and of the downstream opening/closing means. In the embodiment shown in Fig. 1, the liquid medicine pressurizing/supplying means 1 and the secondary pressurizing means 5 are connected to each other by an upstream passage 2 and a downstream passage 6 is provided downstream of the secondary pressurizing means 5.

In the liquid medicine infusion apparatus of the present invention, the liquid medicine pressuring/supplying means 1 stores and forces out the liquid medicine toward the secondary pressuring means 5 by pressurization to cause a flow of the liquid medicine. As the liquid medicine pressuring/supplying means 1, there may be used a balloon which makes use of the shrinkage force of a rubber elastic body. The liquid medicine is stored inside of the expanded balloon. The balloon is shrunk by natural force of the rubber elastic body. A syringe which makes use of the resilient pressure of a spring is also used as the liquid medicine pressuring/supplying means 1. The liquid medicine is stored inside of the syringe and forced out of the syringe using a plunger having a resilient spring. A pressure bag which makes use of air pressure is also used. The pressure bag is made of a flexible plastic film and contained in the hard container. The liquid medicine is stored in the bag and forced out of the bag using an air introduction into the hard container to press the bag. A means that mechanically pressurizes a bag filled with the liquid medicine by a pump, such as infusion pump is also used. As for the pressuring/supplying means 1, a pressure of 50 to 100 kPa is desirably applied to the liquid medicine to force it out toward the secondary pressuring means 5.

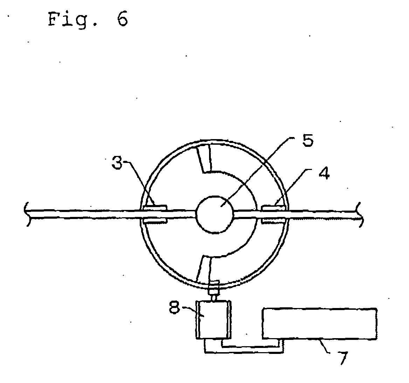

In the liquid medicine infusion apparatus of the present invention, the secondary pressurizing means 5 is connected in liquid communication with the liquid medicine pressurizing/supplying means 1 by the upstream passage 2, temporarily stores the liquid medicine supplied from the liquid medicine pressurizing/supplying means 1, and forces it out toward the downstream side by pressurization. The secondary pressurizing means 5 shown in Fig. 1 consists of a spring 51, a gasket 52, and a cylinder 53, and generates internal pressure by making use of the elastic force of the spring. Other examples of the secondary pressurizing means include those which generate internal pressure by using a plastic sheet 54 such as a vinyl chloride, silicone rubber, or thermoplastic elastomer sheet in place of the gasket and pressing it by the spring 51 as shown in Fig. 3 and those using a rubber elastic balloon as shown in Fig. 4. However, the present invention is not limited to these means. The pressure (internal pressure) of the secondary pressurizing means 5 is generally set to a value 10 to 20% lower than the pressure of the liquid medicine pressurizing/supplying means 1. Therefore, the liquid medicine can flow from the liquid medicine pressurizing/supplying means 1 to the secondary pressurizing means 5.

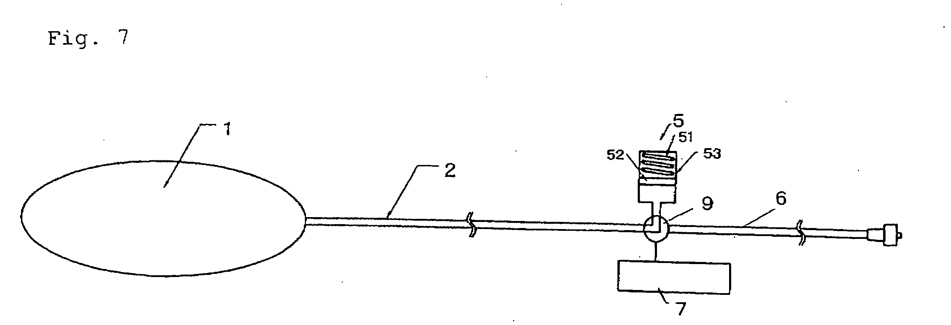

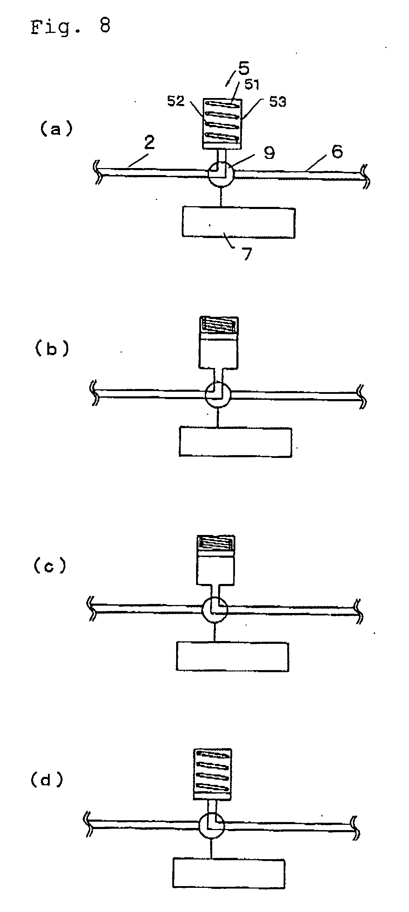

In the liquid medicine infusion apparatus of the present invention, the upstream opening/closing means 3 is arranged in the upstream passage 2 to open or close the liquid communication between the liquid medicine pressurizing/supplying means 1 and the secondary pressurizing means 5. In the liquid medicine infusion apparatus of the present invention, the downstream opening/closing means 4 is arranged in the downstream passage 6 provided downstream of the secondary pressurizing means 5 to open or close the liquid communication between the secondary pressurizing means 5 and the downstream passage 6. It is preferred that the upstream opening/closing means 3 and the downstream opening/closing means 4 can be opened/closed with an operational force as small as possible to allow usage for a long time. The upstream opening/closing means 3 and the downstream opening/closing means 4 are not necessarily separate units and can be integrated into a single unit capable of performing the functions of both means. The upstream opening/closing means 3 and the downstream opening/closing means 4 shown in Fig. 1 are electromagnetic valves. Other examples of the upstream opening/closing means 3 and the downstream opening/closing means 4 include clamps for clamping a tube used as the liquid medicine passage from outside to close the passage at predetermined time intervals by making use of the rotation of a motor 8 shown in Figs. 5 and 6, and an integrated unit obtained by integrating the upstream opening/closing means 3 and the downstream opening/closing means 4 by making use of a stopcock 9 shown in Figs. 7 and 8. However, the present invention is not limited to these means.

The suitable materials of the first and second passages 2 and 6 are thermoplastic tubing made of polyvinyl chloride, polyethylene, polybutadiene, silicone and the like.The suitable inner diameter of the tubing is from about 0.5 mm to about 5mm. The first and second passage 2 and 6 do not restrict the flow of the liquid medicine.

In the liquid medicine infusion apparatus of the present invention, the control means 7 serves to control the opening/closing timing of the upstream opening/closing means 3 and of the downstream opening/closing means 4. The control of the opening/closing timing of these opening/closing means makes it possible to attain a predetermined liquid medicine infusion rate in the liquid medicine infusion apparatus of the present invention.

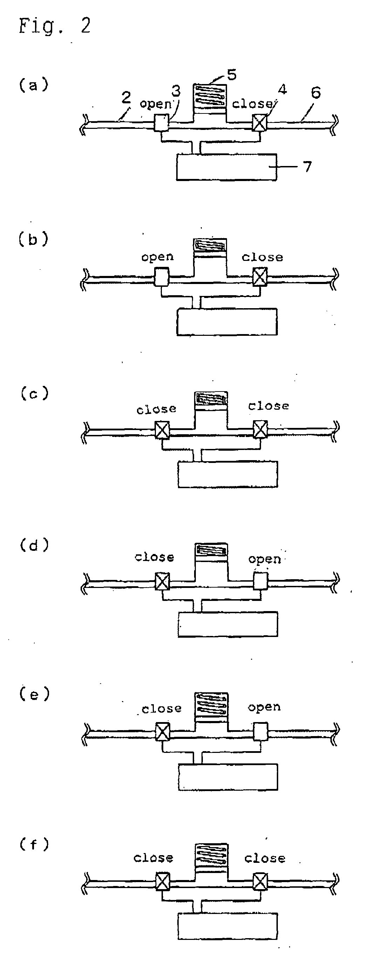

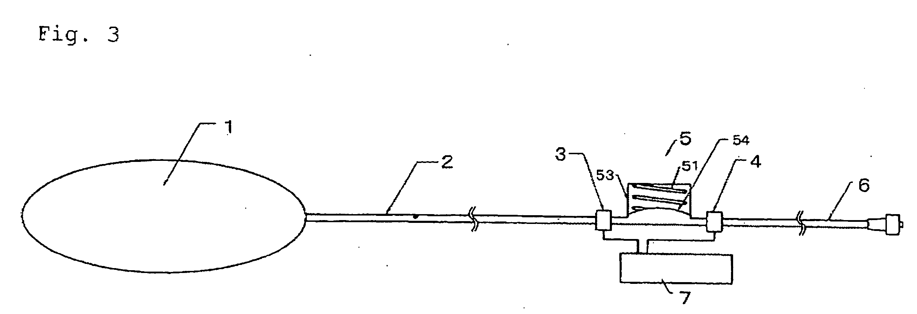

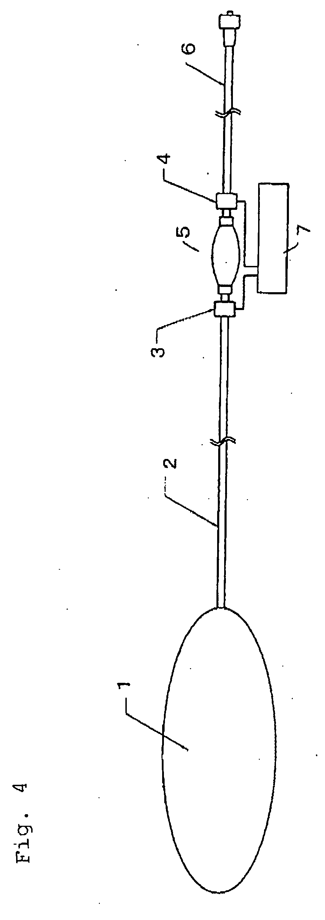

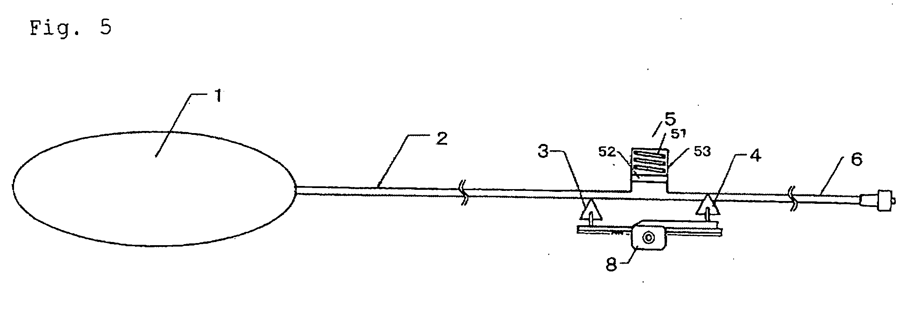

A description is subsequently given of the liquid medicine infusing operation of the liquid medicine infusion apparatus according to the embodiment of the present invention shown in Fig. 1 with reference to Figs. 2(a) to 2(f).

As described above, the liquid medicine infusion apparatus of the present invention infuses a liquid medicine by controlling the opening/closing timing of the upstream opening/closing means 3 and of the downstream opening/closing means 4 by the control means 7.

When the liquid medicine infusion operation is started while the upstream opening/closing means 3 is opened and the downstream opening/closing means 4 is closed (Fig. 2 (a)), because the pressure (internal pressure) of the secondary pressurizing means 5 is set to a value lower than the pressure of the liquid medicine pressurizing/supplying means 1, the liquid medicine flows into the secondary pressurizing means 5 from the liquid medicine pressurizing/supplying means 1 and is stored in the secondary pressurizing means 5. Since the capacity of the secondary pressurizing means 5 is very small, the liquid medicine from the liquid medicine pressurizing/supplying means 1 is filled into the secondary pressurizing means 5 substantially instantaneously after the start of the liquid medicine infusing operation, and the state shown in Fig. 2(b) is obtained.

When the filling of the liquid medicine into the secondary pressurizing means 5 is completed, the upstream opening/closing means 3 is closed, and the state shown in Fig. 2(c) is obtained.

Next, when the downstream opening/closing means 4 is opened (Fig. 2 (d) ) , the liquid medicine stored in the secondary pressurizing means 5 is forced out by the pressure (internal pressure) of the secondary pressurizing means 5 and supplied to a patient through the downstream passage 6. Since the capacity of the secondary pressurizing means 5 is sufficiently small and the pressure of the secondary pressurizing means 5 is applied to the liquid medicine, the liquid medicine stored in the secondary pressurizing means 5 is completely discharged into the downstream passage 6 substantially instantaneously upon opening of the downstream opening/closing means 4, and the state shown in Fig. 2 (e) is obtained.

When the discharge of the liquid medicine from the secondary pressurizing means 5 is completed, the downstream opening/closing means 4 is closed, and the state shown in Fig. 2(f) is obtained.

The above operation of dosing a patient with the liquid medicine little by little intermittently (i.e., intermittently in small increments) is repeated so that the liquid medicine infusion apparatus of the present invention can infuse the liquid medicine stably while maintaining a fixed infusion rate. For example, when the inner capacity of the secondary pressurizing means 5 is 0.05 ml and the liquid medicine infusion operation is repeated once a minute, 0.05 ml per minute or 3 ml per hour of a liquid medicine is dosed to a patient. Even when the inner capacity of the secondary pressurizing means 5 is 0.05 ml, by repeating the liquid medicine infusion operation twice a minute, 0.1 ml per minute or 6 ml per hour of a liquid medicine is dosed to a patient.

Therefore, in the liquid medicine infusion apparatus of the present invention, the liquid medicine infusion rate (dose of the liquid medicine to a patient per unit time) can be controlled by adjusting the opening/closing timing of the upstream opening/closing means and of the downstream opening/closing means by the control means.

Thus, the liquid medicine infusion apparatus of the present invention has an advantage in that a predetermined fixed infusion rate can be maintained even when the viscosity of a liquid medicine changes due to a change in the type or temperature of the liquid medicine because it controls the liquid medicine infusion rate (flow rate) without making use of the line resistance of a small-diameter tube, unlike conventional liquid medicine infusion apparatuss in which liquid medicine pressurizing/supplying means such as a balloon is connected to a flow control unit composed of a small-diameter tube. In addition, unlike conventional liquid medicine infusion apparatuses which generate power for forcing out a liquid medicine by using electric energy, the liquid medicine infusion apparatus of the present invention requires no electric energy, or even if it does, it requires only the electric energy necessary for the control means to open or close the upstream opening/closing means and the downstream opening/closing means. Therefore, the liquid medicine infusion apparatus of the present invention does not require a large battery even when it is used for a long time, thereby making it possible to reduce the weight of a pump itself. Accordingly, the liquid medicine infusion apparatus of the present invention has advantages in that it can be conveniently carried by a patient and its cost can be reduced.

**Claims**

1. A liquid medicine infusion apparatus for infusing a liquid medicine by comprising:

   (a) a liquid medicine pressurizing/supplying means (1) for exerting pressure on the liquid medicine and causing the liquid medicine to flow through a first passage (2);
   (b) a secondary liquid medicine pressurizing means (5) which is connected in liquid communication with the liquid medicine pressurizing/supplying means (1) by said first passage (2) and which is sized to receive an increment of liquid medicine from the liquid medicine pressuring/supplying means (1) and which exerts a pressure on the liquid medicine that is lower than the pressure exerted on the liquid medicine by the liquid medicine pressuring/supplying means (1);
   (c) upstream opening/closing means (3) arranged in the first passage (2), for opening and closing the liquid communication state between the liquid medicine pressurizing/supplying means (1) and the secondary liquid medicine pressurizing means (5);
   (d) downstream opening/closing means (4) arranged in a second passage (6) connected to and provided downstream of the secondary pressurizing means (5); and
   (e) control means (7) for controlling the opening/closing timing of the upstream opening/closing means (3) and of the downstream opening/closing means (4).

2. The liquid medicine infusion apparatus according to claim 1, wherein means for pressurizing liquid medicine of the liquid medicine pressuring/supplying means (1) is a rubber elastic body.

3. The liquid medicine infusion apparatus according to claim 1, wherein means for pressurizing liquid medicine of the liquid medicine pressurizing/supplying means (1) is a spring.

4. The liquid medicine infusion apparatus according to claim 1, wherein means for pressurizing liquid medicine of the liquid medicine pressurizing/supplying means (1) is air pressure.

5. The liquid medicine infusion apparatus according to any one of claims 1-4, wherein means for pressurizing liquid medicine of the secondary liquid medicine pressurizing means (5) is a rubber elastic body.

6. The liquid medicine infusion apparatus according to any one of claims 1-4, wherein means for pressurizing liquid medicine of the secondary liquid medicine pressurizing means (5) is a spring (51).

7. The liquid medicine infusion apparatus according to any one of claims 1-4, wherein means for pressurizing liquid medicine of the secondary liquid medicine pressurizing means (5) is air pressure.

8. The liquid medicine infusion apparatus according to any one of claims 1-7, wherein the upstream opening/closing means (3) and the downstream opening/closing means (4) comprises electromagnetic valves.

9. The liquid medicine infusion apparatus according to any one of claims 1-7, wherein the upstream opening/closing means (3) and the downstream opening/closing means (4) comprises clamps.

10. The liquid medicine infusion apparatus according to any one of claims 1-7, wherein the upstream opening/closing means (3) and the downstream opening/closing means (4) are comprised of an integrated unit using a stopcock (9).

Fig. 1

Fig. 2

(a)

(b)

(c)

(d)

(e)

(f)

Fig. 3

EP 1 410 814 A2

EP 1 410 814 A2

Fig. 4

Fig. 5

Fig. 6

Fig. 7

Fig. 8

(a)

(b)

(c)

(d)